(19) Europäisches Patentamt
European Patent Office
Office européen des brevets

(11) Veröffentlichungsnummer: **0 109 925**
**A1**

# (12) EUROPÄISCHE PATENTANMELDUNG

(21) Anmeldenummer: 83810426.3

(22) Anmeldetag: 26.09.83

(51) Int. Cl.³: C 07 D 233/80
A 01 N 43/50

(30) Priorität: 30.09.82 CH 5753/82

(43) Veröffentlichungstag der Anmeldung:
30.05.84 Patentblatt 84/22

(84) Benannte Vertragsstaaten:
AT BE CH DE FR IT LI LU NL

(71) Anmelder: CIBA-GEIGY AG
Postfach
CH-4002 Basel(CH)

(72) Erfinder: Riebli, Peter
Bünten 17
CH-4446 Buckten(CH)

(72) Erfinder: Hubele, Adolf, Dr.
Obere Egg 9
CH-4312 Magden(CH)

## (54) Mikrobizide Imidazolidin-2,4-dion-Derivate.

(57) Es werden neue Imidazolidin-2,4-dion-Derivate der allgemeinen Formel I beschrieben,

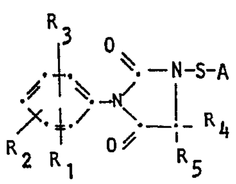

(I)

worin

$R_1$ und $R_2$ unabhängig voneinander für Wasserstoff, Halogen, $C_1$-$C_3$-Alkyl, Nitro, $C_1$-$C_3$-Haloalkyl, $C_1$-$C_3$-Alkoxy, $C_1$-$C_3$-Haloalkoxy, Cyano oder $C_1$-$C_3$-Alkylthio stehen;

$R_3$ Wasserstoff, Halogen oder Methyl bedeutet;

$R_4$ und $R_5$ unabhängig voneinander für Wasserstoff oder $C_1$-$C_3$-Alkyl stehen; und

A eine Haloethylgruppe symbolisiert,

die hervorragende mikrobizide Eigenschaften aufweisen und daher zum Einsatz im Pflanzenschutz prädestiniert sind. Es wird ein Verfahren zur Herstellung dieser Substanzen aufgezeigt, das durch Umsetzung von Verbindungen der Formel II

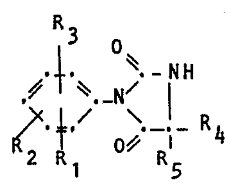

(II)

mit Verbindungen der Formel III C1-S-A gekennzeichnet ist. Ferner werden agrochemische Mittel beschrieben, die mindestens einen dieser neuen Wirkstoffe enthalten, sowie Verfahren zur Bekämpfung wichtiger Pflanzenschädlinge.

EP 0 109 925 A1

Croydon Printing Company Ltd.

0109925



CIBA-GEIGY AG

Basel (Schweiz)

5-14109/=

Mikrobizide Imidazolidin-2,4-dion-Derivate

Die vorliegende Erfindung betrifft neue Imidazolidin-2,4-dion-Derivate der nachstehenden Formel I.
Die Erfindung betrifft auch die Herstellung der neuen Substanzen, agrochemische Mittel die mindestens eine dieser Verbindungen als Wirkstoff enthalten sowie die Herstellung dieser Mittel.
Die Erfindung betrifft ferner ein Verfahren zur Bekämpfung oder präventiven Verhütung eines Befalls von Pflanzen durch schädliche Mikroorganismen.

Die erfindungsgemässen, neuen Verbindungen gehorchen der allgemeinen Formel I,

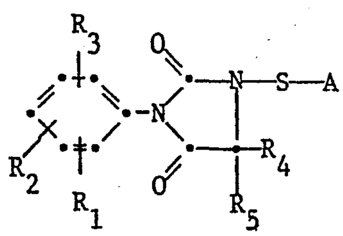

(I),

worin

$R_1$ und $R_2$ unabhängig voneinander für Wasserstoff, Halogen, $C_1$-$C_3$-Alkyl, Nitro, $C_1$-$C_3$-Haloalkyl, $C_1$-$C_3$-Alkoxy, $C_1$-$C_3$-Haloalkoxy, Cyano oder $C_1$-$C_3$-Alkylthio stehen;

$R_3$ Wasserstoff, Halogen oder Methyl bedeutet;

$R_4$ und $R_5$ unabhängig voneinander für Wasserstoff oder $C_1$-$C_3$-Alkyl stehen; und

A eine Haloethylgruppe symbolisiert.

Unter dem Betriff Alkyl selbst oder als Bestandteil eines anderen Substituenten, wie z.B. Haloalkyl, Haloalkoxy, Alkoxy oder Alkylthio, sind je nach Anzahl der angegebenen Kohlenstoffatome beispielsweise folgende geradkettige oder verzweigte aliphatische Gruppen zu verstehen: Methyl, Ethyl, n-Propyl, iso-Propyl usw. Halogen steht hier und im folgenden für Fluor, Chlor, Brom oder Jod, als Substituent im Phenylteil vorzugsweise für Fluor oder Chlor und als Substituent im Haloethylteil A insbesondere für Fluor, Chlor oder Brom. Haloalkyl steht für einen einfach halogenierten bis perhalogenierten Alkylrest, so z.B. eine Halomethylgruppe für $CH_2Cl$, $CHCl_2$, $CCl_3$, $CH_2Br$, $CHBr_2$, $CBr_3$, $CH_2F$, $CHF_2$, bevorzugt $CF_3$, $CH_2J$, $CHJ_2$, $CJ_3$, $CCl_2F$, $CClF_2$ usw.. Haloalkoxy steht für einen einfach halogenierten bis perhalogenierten Alkoxyrest, so z.B. für $OCHF_2$, $OCHFCl$, $OCH_2F$, $OCF_3$, $OCF_2CHFCl$, $OCH_2CF_3$, bevorzugt $OCF_3$, $OCF_2CHF_2$ und $OCH_2CF_3$. Eine Haloethylgruppe steht beispielsweise für eine durch gleiche oder verschiedene Halogenatome substituierte Ethylgruppe wie $CF_2CF_3$, $CCl_2CCl_3$, $CCl_2CCl_2H$, $CHClCHCl_2$, $CHBrCHClBr$, $CF_2CF_2Cl$, $CCl_2CCl_2F$, $CBr_2CBr_3$, $CF_2CF_2H$, $CBr_2CBr_2H$, $CCl_2CH_2Cl$, $CF_2CH_3$, $CH_2CH_2F$ usw. Entsprechend steht eine Halopropylgruppe für eine durch gleiche oder verschiedene Halogenatome substituierte n-Propyl- oder iso-Propylgruppe.

Auf Grund ihrer vorteilhaften pflanzenfungiziden Eigenschaften sind nachfolgende Untergruppen und Einzelsubstanzen zunehmend bevorzugt.

Untergruppe Ia: Verbindungen der Formel I, worin die Substituenten $R_1$ und $R_2$ unabhängig voneinander für Wasserstoff, Fluor, Chlor, Brom, Methyl, Ethyl, Nitro, Trifluormethyl, Methoxy, Ethoxy, Trifluormethoxy, Cyano or Methylthio stehen; $R_3$ Wasserstoff, Fluor, Chlor, Brom oder Methyl bedeutet; $R_4$ und $R_5$ unabhängig voneinander Wasserstoff oder Methyl bedeuten; und A für $CCl_2CHCl_2$, $C_2Cl_5$, $CHBrCHBrCl$, $C_2F_5$, $CF_2CF_2Cl$ oder $CCl_2CCl_2F$ steht.

Untergruppe Ib: Verbindungen der Formel I, worin die Substituenten $R_1$ und $R_2$ unabhängig voneinander für Wasserstoff, Fluor, Chlor, Methyl,

Trifluormethyl, Methoxy, Trifluormethoxy, oder Methylthio stehen; $R_3$ Wasserstoff, Fluor, Chlor, Brom oder Methyl bedeutet; $R_4$ für Wasserstoff steht; $R_5$ Wasserstoff bedeutet; und A für $CCl_2CHCl_2$, $C_2Cl_5$, CHBrCHBrCl, $CF_2CF_2Cl$ oder $CCl_2CCl_2F$ steht.

Untergruppe Ic: Verbindungen der Formel I, worin $R_1$ für Fluor, Chlor, Brom, Methyl, Ethyl, Nitro, Trifluormethyl, Methoxy, Ethoxy, Trifluormethoxy, Cyano oder Methylthio steht; $R_2$ Wasserstoff, Fluor, Chlor, Brom oder Methyl bedeutet; $R_3$ für Wasserstoff, Fluor, Chlor oder Methyl steht; $R_4$ und $R_5$ unabhängig voneinander für Wasserstoff oder Methyl stehen; und A $CCl_2CHCl_2$, $C_2Cl_5$, CHBrCHBrCl, $CF_2CF_2Cl$ oder $CCl_2CCl_2F$ bedeutet.

Innerhalb der Untergruppe Ic sind vor allem jene Verbindungen der Formel I bevorzugt, worin $R_4$ und $R_5$ für Wasserstoff stehen und A für $CCl_2CHCl_2$ steht.

Besonders bevorzugte Einzelsubstanzen sind z.B.:

1-(1',1',2',2'-Tetrachlorethylthio)-3-(3'-chlorphenyl)-imidazolidin-2,4-dion (= Verb. Nr. 1.2)

1-(1',1',2',2'-Tetrachlorethylthio)-3-(4'-methoxyphenyl)-imidazolidin-2,4-dion (=Verb.Nr. 1.7)

1-(1',1',2',2'-Tetrachlorethylthio)-3-(3'-methoxyphenyl)-imidazolidin-2,4-dion (= Verb. Nr. 1.9)

Die neuen Imidazolidin-2,4-dion-Derivate der Formel I lassen sich erfindungsgemäss, entsprechend der nachfolgenden Reaktionsgleichung (α), dadurch herstellen,

(α) 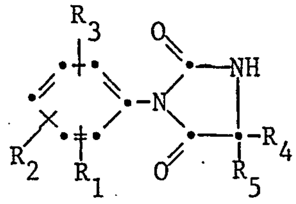 + X - S - A



(II) (III)

dass man eine Verbindung der Formel II, vorzugsweise in Gegenwart eines reaktionsinerten Lösungsmittels oder Lösungsmittelgemisches und

in Anwesenheit eines säurebindenden Mittels mit einer Verbindung der Formel III umsetzt, wobei X für Halogen, bevorzugt Chlor oder Brom steht. Hierbei liegen vorteilhafte Reaktionstemperaturen zwischen -30° und +100°C, vorzugsweise zwischen -20° und +10°C.

Geeignete reaktionsinerte Lösungs- oder Verdünnungsmittel sind z.B. aliphatische und aromatische Kohlenwasserstoffe, wie Petrolether, Hexan, Benzol, Toluol, Xylole usw; halogenierte Kohlenwasserstoffe, wie Chlorbenzol, Methylenchlorid, Chloroform, Tetrachlorkohlenstoff, usw; Nitrobenzol; Ether und etherartige Verbindungen, wie Dialkylether (Diethylether, Diisopropylether, tert.-Butylmethylether usw.), Dioxan, Tetrahydrofuran usw.; wie Ethylacetat, Methylacetat usw. sowie Gemische derartiger Verbindungen untereinander.

Als säurebindende Mittel kommen z.B. anorganische Basen, wie Hydroxide, Carbonate und Hydrogencarbonate von Alkali- und Erdalkalimetallen sowie die Hydride der Alkalimetalle in Frage, so z.B. NaOH, KOH, LiOH, $Mg(OH)_2$, $K_2CO_3$, $Na_2CO_3$, $KHCO_3$, $NaHCO_3$, $CaCO_3$, $MgCO_3$, $BaCO_3$ usw.. Ferner kommen organische Basen in Frage, so z.B. tertiäre Amine, wie Trialkylamine (Triethylamin, Diethylmethylamin, Tripropylamin, Dimethylanilin, Methylethylanilin, usw.), Pyridin und Pyridinbasen, wie 4-Dimethylaminopyridin, 4-Pyrrolidylopyridin usw.

Die Ausgangsverbindungen der Formeln II und III werden im allgemeinen in stöchiometrischen Mengen umgesetzt. In manchen Fällen kann III auch im Ueberschuss zugegeben werden.

Verbindungen der Formel II sind aus der Literatur bekannt, z.B. aus den Deutschen Offenlegungsschriften 1,958,183 und 2,636,549 oder lassen sich nach an sich bekannten Methoden herstellen. Die Substanzen der Formel III sind ebenfalls bekannt, oder nach an sich bekannten Methoden herstellbar.

Mikrobizide Substanzen, insbesondere Pflanzenfungizide mit einem Imidazolidin-2,4-dion-Grundgerüst sind aus der Literatur bekannt. So werden Imidazolidin-2,4-dione der nachstehenden Formel (i) in der Deutschen Offenlegungsschrift 1,958,183 als Mikrobizide beschrieben.

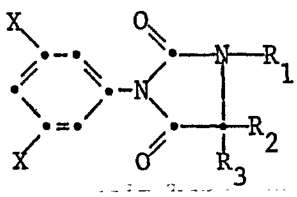

(i)

worin:

X = Halogen.

$R_2$ = H, $C_1$-$C_6$-Alkyl das durch eine Methylenmercatpogruppe substituiert sein kann, Phenyl, Benzyl.

$R_3$ = H, $C_1$-$C_6$-Alkyl, Phenyl.

$R_1$ = H, $C_1$-$C_5$-Alkyl, Phenyl.

In dieser Literaturstelle wird die 3,5-Dihalosubstitution des Phenylrings als zwingende Voraussetzung für eine physiologische Aktivität des Imidazolidin-2,4-diontyps beschrieben (vgl. DE-OS 1,958,183, Seite 3, Absatz 2).

Nunmehr wurde mit dem Auffinden der neuen Imidazolidin-2,4-dione der Formel I überraschenderweise festgestellt, dass eine 3,5-Dihalosubstitution des Phenylrings für eine physiologische Wirkung keine zwingende Voraussetzung darstellt, sondern vielmehr andere Kernsubstitutionen (z.B. in den 2-, 3-, 3,4-, 4-, 2- oder 2,6-Positionen) auch mit weiteren Substituententypen (z.B. $C_1$-$C_3$-Alkyl, Nitro, $C_1$-$C_3$-Alkylthio) ebenfalls zu hervorragenden Pflanzenmikrobiziden führen, so z.B. die Verbindungen Nr. 1.2, 1.7 und 1.9.

Allgemein kann festgestellt werden, dass Verbindungen der Formel I ein für praktische Bedürfnisse sehr günstiges Mikrobizid-Spektrum gegen phytopathogene Pilze und einige Bakterienarten aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Pellicularia, Puccinia); Fungi imperfecti (z.B. Botrytis, Helminthosporium, Fusarium, Septoria, Cercospora, Piricularia und Alternaria) und gegen Phycomyceten wie Pythium, Phytophthora oder Plasmopara. Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytophathogene Pilze eingesetzt werden. Die erfindungsgemässen Wirkstoffe zeichnen sich durch besonders gute Pflanzenverträglichkeit aus. Ihr Haupteinsatzgebiet liegt bei der Bekämpfung von Fungi imperfecti, insbesondere von Botrytis-Arten.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen, sowie die kurative Behandlung befallener Pflanzen oder Flächen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Salat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrere dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen

Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Applikation solcher Mittel kann direkt auf die Pflanze oder Pflanzenteile erfolgen (Blattapplikation) oder auf den Standort der Pflanze (Bodenapplikation) oder auf die Vermehrungsteile, z.B. durch Saatgut-Applikation.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden). Solche Mittel sind gleichfalls Gegenstand vorliegender Erfindung.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethyl-ether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl; oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht sorptive Träger z.B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit gutem Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:

"Mc Cutcheon's Detergents and Emulsifiers Annual" MC Published Corp., Ridgewood New Jersey, 1981.
Helmut Stache "Tensid-Taschenbuch" Carl-Hanser-Verlag, München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei; DMSO = Dimethylsulfoxid; DMF = Dimethylformamid; m.p. = Schmelzpunkt; b.p. Siedepunkt.

Herstellungsbeispiele

Beispiel a:

Synthese von 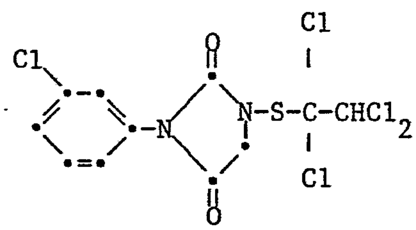

(Verb. Nr. 1.2)

1-(1',1',2',2'-Tetrachlorethylthio)-3-(3'-Chlorphenyl)-imidazolidin-2,4-dion

Zu einer Aufschlämmung von 10,5 g 3-(3'-Chlorphenyl)-imidazolidin-2,4-dion in 100 ml Ethylacetat werden bei -10° bis -5°C unter gutem Rühren innerhalb von 20 Min. 14,1 g 1,1,2,2-Tetrachlorethansulfenylchlorid in 50 ml Ethylacetat zugetropft. Die Mischung wird 1 h bei -5°C weitergerührt, dann wird innerhalb von 30 Min. 5,7 g Triethylamin in 50 ml Ethylacetat zugetropft und das Gemisch anschliessend 16 h bei RT weitergerührt. Das abgeschiedene Triethylaminhydrochlorid wird abfiltriert, mit 70 ml Ethylacetat nachgewaschen, die Waschlösung und das Filtrat vereinigt und das Lösungsmittel im Vakuum verdampft. Der feste Rückstand wird aus Ethanol umkristallisiert. Die weissen Kristalle schmelzen bei 127-130°C.

Beispiel b:

Synthese von

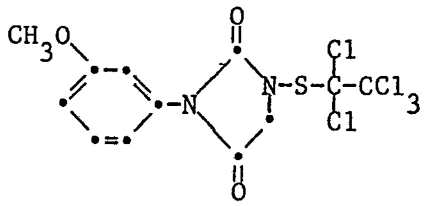

(Verb. Nr. 1.17)

1-(Pentachlorethylthio)-3-(3'-methoxyphenyl)-imidazolidin-2,4-dion

Zu 10,6 g 3-(3'-Methoxyphenyl)-imidazolidin-2,4-dion in 100 ml Ethylacetat werden bei -10° bis -5°C unter gutem Rühren innerhalb von 1,5 h 16,0 g Pentachlorethansulfenylchlorid in 50 ml Ethylacetat zugetropft. Nach 0,5 h bei -5°C werden innerhalb von 30 Min. 5,9 g Triethylamin in 50 ml Ethylacetat zugetropft und das Gemisch anschliessend 48 h bei RT weitergerührt. Die gelbe Suspension wird dann 3 mal mit je 150 ml Wasser gewaschen, die organische Phase über $Na_2SO_4$ getrocknet und das Lösungsmittel verdampft. Zur Reinigung wird das Produkt über eine Kieselgelsäure mittels Ether/Chloroform 1:1 chromatographiert. Die erhaltenen weissen Kristalle schmelzen bei 150-151°C.

Nach Art der vorstehenden Beispiele und der einleitend beschriebenen Herstellungsmethode lassen sich auch die nachfolgend aufgeführten Verbindungen gemäss vorliegender Erfindung herstellen:

Tabelle 1: Verbindungen der Formel I mit $R_4 = R_5 = H$

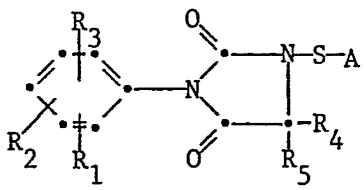

(I)

| Verb. Nr. | $R_1$ | $R_2$ | $R_3$ | A | physikalische Konstante |
|---|---|---|---|---|---|
| 1.1 | H | 4-F | H | $CCl_2CHCl_2$ | m.p. 169-170°C |
| 1.2 | 3-Cl | H | H | $CCl_2CHCl_2$ | m.p. 127-130°C |
| 1.3 | 2-Cl | H | H | $CCl_2CHCl_2$ | |
| 1.4 | 2-F | H | H | $CCl_2CHCl_2$ | |
| 1.5 | $2-CH_3$ | 4-Cl | H | $CCl_2CHCl_2$ | m.p. 149-153°C |
| 1.6 | H | H | H | $CCl_2CHCl_2$ | m.p. 168-170°C |
| 1.7 | H | $4-OCH_3$ | H | $CCl_2CHCl_2$ | m.p. 133-135°C |
| 1.8 | 3-Cl | 4-Cl | H | $CCl_2CHCl_2$ | |
| 1.9 | $3-OCH_3$ | H | H | $CCl_2CHCl_2$ | m.p. 112-114°C |
| 1.10 | 2-Cl | H | 5-Cl | $CCl_2CHCl_2$ | m.p. 145-146°C |
| 1.11 | $2-C_2H_5$ | $6-C_2H_5$ | H | $CCl_2CHCl_2$ | m.p. 68-70°C |
| 1.12 | $2-CH_3$ | H | $6-CH_3$ | $CCl_2CHCl_2$ | |
| 1.13 | $3-CF_3$ | 4-Cl | H | $CCl_2CHCl_2$ | m.p. 133-135°C |
| 1.14 | 3-Cl | $4-CF_3$ | H | $CCl_2CHCl_2$ | m.p. 102-104°C |
| 1.15 | 3-Cl | H | 5-Cl | $CCl_2CHCl_2$ | |
| 1.16 | 2-Cl | 4-Cl | 6-Cl | $CCl_2CHCl_2$ | m.p. 129-130°C |
| 1.17 | $3-OCH_3$ | H | H | $C_2Cl_5$ | m.p. 150-151°C |
| 1.18 | 3-Cl | H | H | $C_2Cl_5$ | |
| 1.19 | H | $4-OCH_3$ | H | $C_2Cl_5$ | |
| 1.20 | $2-CH_3$ | H | $6-CH_3$ | $C_2Cl_5$ | |
| 1.21 | $2-CH_3$ | 4-Cl | H | $C_2Cl_5$ | |
| 1.22 | 2-Cl | H | 5-Cl | $C_2Cl_5$ | |
| 1.23 | H | H | H | $C_2Cl_5$ | |
| 1.24 | H | 4-F | H | CHBrCHBrCl | |
| 1.25 | 3-Cl | H | H | CHBrCHBrCl | |
| 1.26 | H | $4-OCH_3$ | H | CHBrCHBrCl | |
| 1.27 | $3-OCH_3$ | H | H | CHBrCHBrCl | |

Tabelle 1 (Fortsetzung)

| Verb. Nr. | $R_1$ | $R_2$ | $R_3$ | $A^-$ | physikalische Konstante |
|---|---|---|---|---|---|
| 1.28 | 3-Cl | H | 5-Cl | CHBrCHBrCl | |
| 1.29 | 2-$NO_2$ | H | H | $CCl_2CHCl_2$ | |
| 1.30 | 2-CN | H | H | $CCl_2CHCl_2$ | |
| 1.31 | 3-CN | H | H | $CCl_2CHCl_2$ | |
| 1.32 | 3-$CH_3$ | H | H | $CCl_2CHCl_2$ | m.p. 162-164°C |
| 1.33 | H | 4-$CH_3$ | H | $CCl_2CHCl_2$ | m.p. 171-172°C |
| 1.34 | H | 4-Cl | H | $CCl_2CHCl_2$ | m.p. 169-172°C |
| 1.35 | 2-$OCH_3$ | H | H | $CCl_2CHCl_2$ | |
| 1.36 | 3-F | H | H | $CCl_2CHCl_2$ | |
| 1.37 | H | 4-$SCH_3$ | H | $CCl_2CHCl_2$ | m.p. 134-137°C |
| 1.38 | 2-$CF_3$ | H | H | $CCl_2CHCl_2$ | |
| 1.39 | 3-$CF_3$ | H | H | $CCl_2CHCl_2$ | |
| 1.40 | H | 4-$CF_3$ | H | $CCl_2CHCl_2$ | |
| 1.41 | H | 4-$OCH_3$ | H | $C_2F_5$ | |
| 1.42 | H | 4-$OCH_3$ | H | $CF_2CF_2Cl$ | |
| 1.43 | H | 4-$SCH_3$ | H | $CF_2CF_2Cl$ | |
| 1.44 | 3-$OCH_3$ | H | H | $CF_2CF_2Cl$ | |
| 1.45 | 3-$OCH_3$ | H | H | $CCl_2CCl_2F$ | |
| 1.46 | 3-Cl | H | H | $CCl_2CCl_2F$ | |
| 1.47 | 2-$CF_3$ | H | H | $CCl_2CCl_2F$ | |
| 1.48 | 3-$CF_3$ | H | H | $CCl_2CCl_2F$ | |
| 1.49 | 3-$OCH_3$ | H | H | $C_2F_5$ | |
| 1.50 | 3-$SCH_3$ | H | H | $CCl_2CCl_2F$ | |
| 1.51 | H | 4-$OCF_3$ | H | $CCl_2CHCl_2$ | m.p. 123-126°C |
| 1.52 | H | 4-$OCHF_2$ | H | $CCl_2CHCl_2$ | |
| 1.53 | H | 4-$OCF_2CHF_2$ | H | $CCl_2CHCl_2$ | |
| 1.54 | 2-$CH_3$ | 4-$OCH_2CF_3$ | H | $CCl_2CHCl_2$ | |
| 1.55 | 2-$CH_3$ | 4-$OCF_2CHF_2$ | H | $CCl_2CHCl_2$ | |
| 1.56 | H | 4-$OCF_2CHFCl$ | H | $CCl_2CHCl_2$ | |

Tabelle 2: Verbindungen der Formel I

| Verb. Nr. | $R_1$ | $R_2$ | $R_3$ | A | $R_4$ | $R_5$ | Physikalische Konstante |
|---|---|---|---|---|---|---|---|
| 2.1 | 3-Cl | 4-Cl | H | $CCl_2CHCl_2$ | $CH_3$ | H | m.p. 97-99°C |
| 2.2 | 3-Cl | H | 5-Cl | $CCl_2CHCl_2$ | $CH_3$ | H | m.p. 101-103°C |
| 2.3 | 3-$OCH_3$ | H | H | $CCl_2CHCl_2$ | $CH_3$ | H | |
| 2.4 | 3-$OCH_3$ | H | H | $CCl_2CHCl_2$ | $CH_3$ | $CH_3$ | |
| 2.5 | 3-$CF_3$ | H | H | $CCl_2CHCl_2$ | $CH_3$ | H | |
| 2.6 | H | 4-$OCH_3$ | H | $C_2Cl_5$ | $C_2H_5$ | $C_2H_5$ | |
| 2.7 | 3-$OCH_3$ | H | H | $C_2Cl_5$ | $CH_3$ | H | |
| 2.8 | 3-$CF_3$ | H | H | $C_2F_5$ | $CH_3$ | $CH_3$ | |
| 2.9 | 3-$CF_3$ | 4-$CF_3$ | H | CHBrCHBrCl | $CH_3$ | H | |
| 2.10 | 3-Cl | H | H | CHBrCHBrCl | $CH_3$ | $CH_3$ | |
| 2.11 | 3-$OCH_3$ | H | H | CHBrCHBrCl | $C_2H_5$ | H | |
| 2.12 | H | 4-Cl | H | CHBrCHBrCl | $CH_3$ | $C_3H_7$-i | |
| 2.13 | H | 4-$CF_3$ | H | $CCl_2CCl_2F$ | $CH_3$ | H | |
| 2.14 | H | 4-F | H | $CCl_2CCl_2F$ | $CH_3$ | H | |
| 2.15 | 3-$OCH_3$ | H | H | $CCl_2CCl_2F$ | $CH_3$ | $CH_3$ | |
| 2.16 | 3-Cl | H | 5-Cl | $CCl_2CCl_2F$ | $CH_3$ | H | |
| 2.17 | 3-$OCH_3$ | H | H | $CCl_2CHCl_2$ | $C_2H_5$ | H | |
| 2.18 | H | 4-Cl | 5-Cl | $CCl_2CHCl_2$ | $C_3H_7$-i | H | |
| 2.19 | 3-$OCH_3$ | H | H | $CCl_2CHCl_2$ | $C_3H_7$-n | H | |
| 2.20 | 3-$OCH_3$ | 4-$OCH_3$ | H | $CCl_2CHCl_2$ | $C_3H_7$-n | $C_3H_7$-n | |
| 2.21 | 3-$SCH_3$ | H | H | CHBrCHBrCl | $CH_3$ | H | |
| 2.22 | 3-$CF_3$ | 4-$CF_3$ | H | $CCl_2CCl_2F$ | $CH_3$ | H | |
| 2.23 | 3-$CF_3$ | H | H | $C_2Cl_5$ | $CH_3$ | H | |
| 2.24 | 3-$SCH_3$ | H | H | $CCl_2CCl_2F$ | $CH_3$ | H | |

Formulierungsbeispiele für Wirkstoffe der Formel I

(% = Gewichtsprozent)

| A. Emulsions-Konzentrate | a) | b) | c) |
|---|---|---|---|
| Wirkstoffe aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 20% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentrationen hergestellt werden.

| B. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| C. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| D. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

| E. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

## Biologische Beispiele

### Beispiel B1: Wirkung gegen Erysiphe graminis auf Gerste Residual-protektive Wirkung

Ca 8 cm hohe Gerstenpflanzen wurden mit einer aus Spritzpulver des Wirkstoffs, wie oben beschrieben hergestellten Spritzbrühe (0,02% Aktivsubstanz wie z.B. eine der Verbindungen aus Tabelle 1 und 2

besprüht. Nach 3-4 Stunden wurden die behandelten Pflanzen mit Konidien des Pilzes bestäubt. Die infizierten Gerstenpflanzen wurden im Gewächshaus bei ca. 22°C aufgestellt und der Pilzbefall nach 10 Tagen beurteilt. Dabei wirkten Spritzbrühen, die als Wirkkomponente einen Wirkstoff der Formel I enthielten, z.B. Verbindung Nr. 1.5, 1.6, 1.10, 1.33 und 2.1 eine fast vollständige Hemmung (0-10%) des Befalls, im Vergleich zu unbehandelten Kontrollpflanzen (100% Befall).

Beispiel B2: Wirkung gegen Venturia inaequalis auf Apfel
Residual-protektive Wirkung

Apfelsämlinge mit ca. 5 entwickelten Blättern wurden mit einer aus Spritzpulver, nach einem der obigen Beispiele hergestellten Spritzbrühe (0,06% Aktivsubstanz, wie z.B. eine der Verbindungen aus den Tabellen 1 bis 5) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt, dabei hemmten Spritzbrühen, die als Aktivsubstanz eine der Verbindungen aus den Tabellen 1 und 2 (z.B. Verbindung Nr. 1.5, 1.6, 1.7, 1.9, 1.10, 1.11, 1.13, 1.33, 1.34, 1.37, 1.51 und 2.1) enthielten, den Pilzbefall fast vollständig (0-5% Befall). Befall der Kontrollpflanzen 100%.

Beispiel B3: Wirkung gegen Phytophthora infestans auf Tomatenpflanzen
Residual-protektive Wirkung

Tomatenpflanzen wurden nach 3-wöchiger Anzucht mit einer aus Spritzpulver des Wirkstoffes, wie oben beschrieben hergestellten Spritzbrühe (0,02% Aktivsubstanz, wie z.B. eine der Verbindungen aus den Tabellen 1 bis 5) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% Luftfeuchtigkeit und 20°C.

Die Verbindungen der Formel I zeigten in obigem Versuch gegenüber Phytophtora-Erregern eine ausgeprägte residual-protektive Wirkung. Sie hemmten den Befall auf weniger als 20%. Bei Applikation folgender Verbindungen wurde ein Krankheitsbefall vollständig verhütet: Nr. 1.2, 1.7, 1.10, 1.16, 1.33, 1.34, 1.37, 1.51 und 2.2.

Beispiel B4: Wirkung gegen Piricularia oryzae auf Reispflanzen

Residual-protektive Wirkung

Reispflanzen wurden nach zweiwöchiger Anzucht mit einer aus Spritzpulver des Wirkstoffes hergestellten Spritzbrühe (0,02% Aktibsubstanz) besprüht. Nach 48 Stunden wurden die behandelten Pflanzen mit einer Konidiensuspension des Pilzes infiziert. Nach 5 Tagen Inkubation bei 95-100% relativer Luftfeuchtigkeit und 24°C wurde der Pilzbefall beurteilt.

Reispflanzen, die mit einer Spritzbrühe behandelt worden sind, die als Aktivsubstanz eine der Verbindungen Nr. 1.11 oder 2.2 enthielt, zeigten im Vergleich zu unbehandelten Kontrollpflanzen (100% Befall) weniger als 10% Pilzbefall.

Beispiel B5: Wirkung gegen Cercospora arachidicola auf Erdnusspflanzen

Residual-protektive Wirkung

10-15 cm hohe Erdnusspflanzen wurden mit einer aus Spritzpulver der Wirksubstanz hergestellten Spritzbrühe (0,006 Aktivsubstanz) besprüht une 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen,

die mit Wirkstoffen aus den Tabellen 1 und 2 behandelt wurden, einen stark reduzierten Cercospora-Befall. So verhinderten die Verbindungen 1.2, 1.5, 1.6, 1.7, 1.9, 1.37, 1.51 und 2.1 obigem Versuch das Auftreten von Flecken fast vollständig (0 bis 16%).

Beispiel 36: Wirkung gegen Botrytis cinerea auf Bohnen

Residual-protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100% relativer Luftfeuchtigkeit und 21°C erfolgte die Beurteilung des Pilzbefalls. Die Verbindungen aus den Tabellen 1 und 2 hemmten in vielen Fällen die Pilzinfektion sehr stark. Bei einer Konzentration von 0,02% erwiesen sich z.B. die Verbindungen Nr. 1.1, 1.2, 1.5, 1.6, 1.7, 1.9, 1.10, 1.11, 1.12, 1.13, 1.14, 1.16, 1.17, 1.33, 1.34, 1.37, 1.51, 2.1 und 2.2 als voll wirksam. Der Kranheitsbefall lag bei 0 bis 8%.
Der Botrytis-Befall unbehandelter aber infizierter Bohnenpflanzen betrug 100%.

Patentansprüche für die Vertragsstaaten: BE, DE, FR, IT, LU, NL, CH, LI

1. Imidazolidin-2,4-dion-Derivate der allgemeinen Formel I,

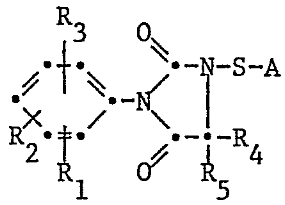

(I)

worin

$R_1$ und $R_2$ unabhängig voneinander für Wasserstoff, Halogen, $C_1$-$C_3$-Alkyl, Nitro, $C_1$-$C_3$-Haloalkyl, $C_1$-$C_3$-Alkoxy, $C_1$-$C_3$-Haloalkoxy, Cyano oder $C_1$-$C_3$-Alkylthio stehen;

$R_3$ Wasserstoff, Halogen oder Methyl bedeutet;

$R_4$ und $R_5$ unabhängig voneinander für Wasserstoff oder $C_1$-$C_3$-Alkyl stehen; und

A eine Haloethylgruppe symbolisiert.

2. Verbindungen der Formel I nach Anspruch 1, worin $R_1$ und $R_2$ unabhängig voneinander für Wasserstoff, Fluor, Chlor, Methyl, Ethyl, Nitro, Trifluormethyl, Methoxy, Ethoxy, Trifluormethoxy, Cyano oder Methylthio stehen; $R_3$ Wasserstoff, Fluor, Chlor, Brom oder Methyl bedeutet; $R_4$ und $R_5$ unabhängig voneinander Wasserstoff oder Methyl bedeuten und A für $CCl_2CHCl_2$, $C_2Cl_5$, CHBrCHBrCl, $C_2F_5$, $CF_2CF_2Cl$ oder $CCl_2CCl_2F$ steht.

3. Verbindungen der Formel I nach Anspruch 1, worin die Substituenten $R_1$ und $R_2$ unabhängig voneinander für Wasserstoff, Fluor, Chlor, Methyl, Trifluormethyl, Methoxy, Trifluormethoxy oder Methylthio stehen; $R_3$ Wasserstoff, Fluor, Chlor, Brom oder Methyl bedeutet; $R_4$ für Wasserstoff steht; $R_5$ Wasserstoff oder Methyl bedeutet; und A für $CCl_2CHCl_2$, $C_2Cl_5$, CHBrCHBrCl, $CF_2CF_2Cl$ oder $CCl_2CCl_2F$ steht.

4. Verbindungen der Formel I nach Anspruch 2, worin $R_1$ Fluor, Chlor, Brom, Methyl, Ethyl, Nitro, Trifluormethyl, Methoxy, Ethoxy, Trifluormethoxy, Cyano oder Methylthio steht; $R_2$ Wasserstoff, Fluor, Chlor,

Brom oder Methyl bedeuten; $R_3$ für Wasserstoff, Fluor, Chlor oder Methyl steht; $R_4$ und $R_5$ unabhängig voneinander für Wasserstoff oder Methyl stehen; und A $CCl_2CHCl_2$, $C_2Cl_5$, CHBrCHBrCl, $CF_2CF_2Cl$ oder $CCl_2CCl_2F$ bedeutet.

5. Verbindungen der Formel I nach Anspruch 4, worin die Substituenten $R_1$, $R_2$ und $R_3$ wie in Anspruch 4 definiert sind; $R_4$ und $R_5$ Wasserstoff bedeuten; und A für $CCl_2CHCl_2$ steht.

6. Eine Verbindung der Formel I, ausgewählt aus der Reihe:

1-(1',1',2',2'-Tetrachlorethylthio)-3-(3'-chlorphenyl)-imidazolidin-2,4-dion;

1-(1',1',2',2'-Tetrachlorethylthio)-3-(4'-methoxyphenyl)-imidazolidin-2,4-dion;

1-(1',1',2',2'-Tetrachlorethylthio)-3-(3'-methoxyphenyl)-imidazolidin-2,4-dion.

7. Verfahren zur Herstellung von Imidazolidin-2,4-dionderivaten der allgemeinen Formel I,

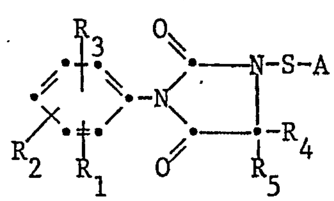

(I)

worin

$R_1$ und $R_2$ unabhängig voneinander für Wasserstoff, Halogen, $C_1$-$C_3$-Alkyl, Nitro, $C_1$-$C_3$-Haloalkyl, $C_1$-$C_3$-Alkoxy, $C_1$-$C_3$-Haloalkoxy, Cyano oder $C_1$-$C_3$-Alkylthio stehen;

$R_3$ Wasserstoff, Halogen oder Methyl bedeutet;

$R_4$ und $R_5$ unabhängig voneinander für Wasserstoff oder $C_1$-$C_3$-Alkyl stehen; und

A eine Haloethylgruppe symbolisiert,

dadurch gekennzeichnet, dass man eine Verbindung der Formel II

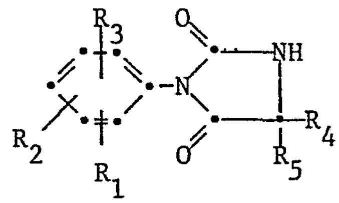

(II)

in Gegenwart eines säurebindenden Mittels mit einer Verbindung der Formel III

$$X - S - A \qquad (III)$$

umsetzt, wobei die Substituenten $R_1$, $R_2$, $R_3$, $R_4$ und $R_5$ in Formel II und A in Formel III die unter Formel I angegebenen Bedeutungen haben und X für Halogen steht.

8. Verfahren nach Anspruch 7, dadurch gekennzeichnet, dass man die Reaktion in einem reaktionsinerten Lösungsmittel oder Lösungsmittelgemisch durchführt.

9. Verfahren nach einem der Ansprüche 7 oder 8, dadurch gekennzeichnet, dass man die Reaktion bei Temperaturen zwischen -30° und +10°C durchführt.

10. Verfahren nach Anspruch 9, dadurch gekennzeichnet, dass man die Reaktion bei Temperaturen zwischen -20° und +10°C durchführt.

11. Mikrobizides Mittel zur Bekämpfung oder Verhütung eines Befalls durch Mikroorganismen, dadurch gekennzeichnet, dass es als mindestens eine aktive Komponente eine Verbindung der Formel I gemäss einem der Ansprüche 1 bis 6 zusammen mit geeigneten Trägerstoffen enthält.

12. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 6 auf die befallenen Flächen, Pflanzen oder den Standort der Pflanzen appliziert.

13. Verfahren gemäss Anspruch 12, dadurch gekennzeichnet, dass es sich bei den Mikroorganismen um phytopathogene Pilze handelt.

14. Verfahren gemäss Anspruch 13, dadurch gekennzeichnet, dass die Pilze der Klasse der Oomyceten, Ascomyceten oder Fungi imperfecti angehören.

15. Verfahren zur Herstellung eines mikrobiziden Mittels gemäss Anspruch 11, dadurch gekennzeichnet, dass mindestens eine der in Anspruch 1 definierten Verbindungen der Formel I mit geeigneten festen und/oder flüssigen Trägerstoffen und/oder Tensiden innig vermischt wird.

Patentansprüche für den Vertragsstaat AT

1. Mikrobizides Mittel, enthaltend als mindestens eine Wirkkomponente ein Imidazolidin-2,4-dion-Derivat der allgemeinen Formel I zusammen mit geeigneten Träger- und Zuschlagstoffen,

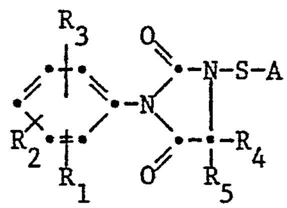 (I)

worin

$R_1$ und $R_2$ unabhängig voneinander für Wasserstoff, Halogen, $C_1$-$C_3$-Alkyl, Nitro, $C_1$-$C_3$-Haloalkyl, $C_1$-$C_3$-Alkoxy, $C_1$-$C_3$-Haloalkoxy, Cyano oder $C_1$-$C_3$-Alkylthio stehen;

$R_3$ Wasserstoff, Halogen oder Methyl bedeutet;

$R_4$ und $R_5$ unabhängig voneinander für Wasserstoff oder $C_1$-$C_3$-Alkyl stehen; und

A eine Haloethylgruppe symbolisiert.

2. Mittel nach Anspruch 1, enthaltend eine Verbindung der Formel I, worin $R_1$ und $R_2$ unabhängig voneinander für Wasserstoff, Fluor, Chlor, Methyl, Ethyl, Nitro, Trifluormethyl, Methoxy, Ethoxy, Trifluormethoxy, Cyano oder Methylthio stehen; $R_3$ Wasserstoff, Fluor, Chlor, Brom oder Methyl bedeutet; $R_4$ und $R_5$ unabhängig voneinander Wasserstoff oder Methyl bedeuten und A für $CCl_2CHCl_2$, $C_2Cl_5$, CHBrCHBrCl, $C_2F_5$, $CF_2CF_2Cl$ oder $CCl_2CCl_2F$ steht.

3. Mittel nach Anspruch 1, enthaltend eine Verbindung der Formel I, worin die Substituenten $R_1$ und $R_2$ unabhängig voneinander für Wasserstoff, Fluor, Chlor, Methyl, Trifluormethyl, Methoxy, Trifluormethoxy oder Methylthio stehen; $R_3$ Wasserstoff, Fluor, Chlor, Brom oder Methyl bedeutet; $R_4$ für Wasserstoff steht; $R_5$ Wasserstoff oder Methyl bedeutet; und A für $CCl_2CHCl_2$, $C_2Cl_5$, CHBrCHBrCl, $CF_2CF_2Cl$ oder $CCl_2CCl_2F$ steht.

4. Mittel nach Anspruch 2, enthaltend eine Verbindung der Formel I, worin $R_1$ Fluor, Chlor, Brom, Methyl, Ethyl, Nitro, Trifluormethyl, Methoxy, Ethoxy, Trifluormethoxy, Cyano oder Methylthio steht; $R_2$ Wasserstoff, Fluor, Chlor, Brom oder Methyl bedeuten; $R_3$ für Wasserstoff, Fluor, Chlor oder Methyl steht; $R_4$ und $R_5$ unabhängig voneinander für Wasserstoff oder Methyl stehen; und A $CCl_2CHCl_2$, $C_2Cl_5$, CHBrCHBrCl, $CF_2CF_2Cl$ oder $CCl_2CCl_2F$ bedeutet.

5. Mittel nach Anspruch 4, enthaltend eine Verbindung der Formel I, worin die Substituenten $R_1$, $R_2$ und $R_3$ wie in Anspruch 4 definiert sind; $R_4$ und $R_5$ Wasserstoff bedeuten; und A für $CCl_2CHCl_2$ steht.

6. Mittel nach Anspruch 1, enthaltend eine Verbindung der Formel I, ausgewählt aus der Reihe:

1-(1',1',2',2'-Tetrachlorethylthio)-3-(3'-chlorphenyl)-imidazolidin-2,4-dion;

1-(1',1',2',2'-Tetrachlorethylthio)-3-(4'-methoxyphenyl)-imidazolidin-2,4-dion;

1-(1',1',2',2'-Tetrachlorethylthio)-3-(3'-methoxyphenyl)-imidazolidin-2,4-dion.

7. Verfahren zur Herstellung von Imidazolidin-2,4-dionderivaten der allgemeinen Formel I,

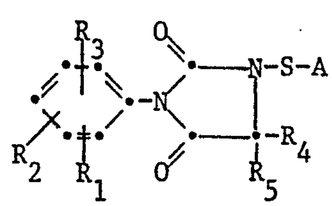

(I)

worin

$R_1$ und $R_2$ unabhängig voneinander für Wasserstoff, Halogen, $C_1$-$C_3$-Alkyl, Nitro, $C_1$-$C_3$-Haloalkyl, $C_1$-$C_3$-Alkoxy, $C_1$-$C_3$-Haloalkoxy, Cyano oder $C_1$-$C_3$-Alkylthio stehen;

$R_3$ Wasserstoff, Halogen oder Methyl bedeutet;

$R_4$ und $R_5$ unabhängig voneinander für Wasserstoff oder $C_1$-$C_3$-Alkyl stehen; und

A eine Haloethylgruppe symbolisiert,
dadurch gekennzeichnet, dass man eine Verbindung der Formel II

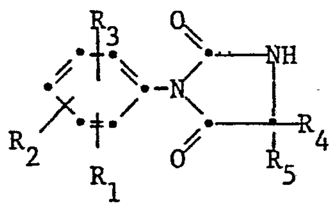

(II)

in Gegenwart eines säurebindenden Mittels mit einer Verbindung der Formel III

X - S - A (III)

umsetzt, wobei die Substituenten $R_1$, $R_2$, $R_3$, $R_4$ und $R_5$ in Formel II und A in Formel III die unter Formel I angegebenen Bedeutungen haben und X für Halogen steht.

8. Verfahren nach Anspruch 7, dadurch gekennzeichnet, dass man die Reaktion in einem reaktionsinerten Lösungsmittel oder Lösungsmittelgemisch durchführt.

9. Verfahren nach einem der Ansprüche 7 oder 8, dadurch gekennzeichnet, dass man die Reaktion bei Temperaturen zwischen -30° und +10°C durchführt.

10. Verfahren nach Anspruch 9, dadurch gekennzeichnet, dass man die Reaktion bei Temperaturen zwischen -20° und +10°C durchführt.

11. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 6 auf die befallenen Flächen, Pflanzen oder den Standort der Pflanzen appliziert.

12. Verfahren gemäss Anspruch 11, dadurch gekennzeichnet, dass es sich bei den Mikroorganismen um phytopathogene Pilze handelt.

13. Verfahren gemäss Anspruch 12, dadurch gekennzeichnet, dass die Pilze der Klasse der Oomyceten, Ascomyceten oder Fungi imperfecti angehören.

14. Verfahren zur Herstellung eines mikrobiziden Mittels, dadurch gekennzeichnet, dass mindestens eine der in Anspruch 1 definierten Verbindungen der Formel I mit geeigneten festen und/oder flüssigen Trägerstoffen und/oder Tensiden innig vermischt wird.

FO 7.5/HL/am*

0109925

Europäisches Patentamt

# EUROPÄISCHER RECHERCHENBERICHT

Nummer der Anmeldung

EP 83 81 0426

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-1 542 710 (CHEVRON) --- | | C 07 D 233/80<br>A 01 N 43/50 |
| A | DE-A-2 441 601 (CIBA-GEIGY) --- | | |
| A | US-A-3 178 447 (KOHN) ----- | | |
| | | | RECHERCHIERTE SACHGEBIETE (Int. Cl. 3) |
| | | | C 07 D 233/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 14-02-1984 | DE BUYSER I.A.F. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82